# EUROPEAN PATENT SPECIFICATION

(11) **EP 2 736 486 B1**
(45) Date of publication and mention of the grant of the patent: **06.03.2019**
(21) Application number: 12741448.0
(22) Date of filing: 23.07.2012
(51) Int. Cl.: A61K 9/00, A61L 26/00

(54) **WOUND-HEALING COMPOSITIONS AND METHOD OF USE**
WUNDHEILENDE ZUSAMMENSETZUNGEN UND VERWENDUNGSVERFAHREN DAFÜR
COMPOSITIONS CICATRISANTES ET LEUR MÉTHODE D'UTILISATION

(30) Priority: 28.07.2011 US 201161512655 P
(43) Date of publication of application: 04.06.2014
(73) Proprietor: 3M Innovative Properties Company, St. Paul, MN 55133-3427 (US); Regents of the University of Minnesota, Minneapolis, MN 55455 (US)
(72) Inventor: PARKS, Patrick, J., Saint Paul, Minnesota 55133-3427 (US); PETERSON, Marnie L., Minneapolis, Minnesota 55455 (US); ANDERSON, Michele J., Minneapolis, Minnesota 55455 (US)
(74) Representative: Vossius & Partner Patentanwälte Rechtsanwälte mbB
(86) International application number: PCT/US2012/047786
(87) International publication number: WO 2013/016255

(56) References cited:
- WO-A1-03/045366
- WO-A1-2004/057965
- WO-A2-94/11010
- WO-A2-2007/143586
- DE-A1- 3 416 777
- Anonymous: "Pharmaceutical compounding and dispensing", 2006, XP002685124, page 247
- Anonymous: "Handbook of pharmaceutical excipients", 2006, XP002685125, pages 66-68
- BENJAMIN A. LIPSKY ET AL: "Topical Antimicrobial Therapy for Treating Chronic Wounds", CLINICAL INFECTIOUS DISEASES, vol. 49, no. 10, 15 November 2009 (2009-11-15), pages 1541-1549, XP55039396, ISSN: 1058-4838, DOI: 10.1086/644732

## Description

The present invention provides a composition comprising an antimicrobial biguanide compound, useful for treating a wound, and a wound dressing comprising the composition and a support.

### BACKGROUND

Compositions of aqueous oak bark extract, and synthetic compositions containing the key active ingredients of oak bark extract, have been disclosed for the treatment of a variety of skin conditions, including fungal infections, minor infections, insect bites, minor burns, sunburn, poison oak, poison ivy, poison sumac, wound healing, pyodermas, dermatitis, pruritic dermatoses, eczema, decubitus ulcers, tropical ulcers, decubitus, psoriasis, impetigo, Kaposi sarcoma, warts, gangrene, ischemic ulcer, keratosis, and skin cancer.

Oak bark extract has been described in U.S. Patent No. 5,080,900 for use in the treatment of skin ulcers, particularly decubitus ulcers or bed sores. This material in a base of WHITFIELD pharmaceutical ointment has also been sold under the trade name BENCELOK® for use in the treatment of minor skin irritations (Whitfield and Bencelok are trademarks for pharmaceutical ointments). The amount of oak bark extract in these materials was relatively low, however. For example, the BENCELOK® preparations have contained from 0.25 to 3% by weight of ash-derived components based upon the total weight of the preparation. WO 03/045366 A1 describe compositions for the treatment of wounds such as diabetic ulcers comprising zinc ions, calcium ions, rubidium ions and/or potassium ions in a pharmaceutically acceptable carrier.

Certain skin wounds fail to maintain a progression of biological events that ultimately lead to healing of the wounds. These skin wounds are designated as "stalled wounds". The fundamental cause(s) of this lack of healing is not well-understood. There exists a need for a treatment for stalled wounds.

### SUMMARY

In general, the invention relates to the treatment of skin wounds (e.g., surgical wounds, pressure ulcers, incisions, abrasions, and the like). In particular, the present invention relates to compositions and wound dressings as defined in the claims.

The inventive compositions comprise an antimicrobial biguanide compound and an active ingredient of inorganic solids comprising salts of potassium, zinc, calcium, and rubidium. The inventive compositions, which can be used alone or in combination with a wound dressing, directly and/or indirectly facilitate biological processes associated with wound healing. In the inventive compositions, the antimicrobial biguanide compound comprises polyhexamethylene biguanide (PHMB).

Herein also described is a composition which can comprise a pharmaceutically acceptable carrier, an effective amount of an active ingredient of inorganic solids, and a biguanide compound comprising an amount effective to reduce the number of viable microorganisms at a wound site. The active ingredient can comprise a potassium salt, a zinc salt, a calcium salt, and a rubidium salt, wherein each of the salts comprises a pharmaceutically-acceptable anion.

In any embodiment of the composition, the active ingredient of inorganic solids can comprise 10-80 parts of potassium ions, 0.00001-20 parts of zinc ions, 0.01-10 parts of calcium ions and rubidium ions in an amount up to 40 parts, said parts being expressed as parts by weight of inorganic solids. In any of the above embodiments, the biguanide compound can be selected from the group consisting of polyhexamethylene biguanide, chlorhexidine, octenidine, derivatives thereof, and combinations thereof. In any of the above embodiments, relative to a composition without the biguanide component, the composition with the biguanide compound can suppress the accumulation of a polypeptide associated with inflammation. In some embodiments, relative to a composition without the active ingredient, the composition with the active ingredient suppresses the accumulation of a polypeptide associated with inflammation.

Herein also described is a wound dressing. The wound dressing can comprise any of the above embodiments of the composition and a support. In any embodiment of the dressing, the support can comprise a fibrous material, a film, a gel, a foam, a hydrocolloid, an alginate, a hydrogel a polysaccharide paste, a plurality of granules, a plurality of beads or a combination of any two or more of the foregoing.

Herein also described is a method of treating a wound. The method can comprise contacting a wound with any of the above embodiments of the composition.

Herein also described is a method of treating a wound. The method can comprise contacting a wound with any of the above embodiments of the wound dressing.

The words "preferred" and "preferably" refer to embodiments described herein that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

A "skin wound", as used herein, refers to a break in the continuity of the skin barrier that may result from trauma (e.g., a puncture, a laceration, or an abrasion) or surgery, for example.

A "chronic wound", non-healing wound", "slow to heal wound", or "stalled wound", as used herein, refers to a category of wound that fails to heal over a typical (e.g., 8-12 weeks) timeframe from inception of the wound to complete closure of the skin at the wound site.

The terms "comprises" and variations thereof do not have a limiting meaning where these terms appear in the description and claims.

As used herein, "a," "an," "the," "at least one," and "one or more" are used interchangeably. Thus, for example, a polypeptide can be interpreted to mean "one or more" polypeptides.

The term "and/or" means one or all of the listed elements or a combination of any two or more of the listed elements.

Also herein, the recitations of numerical ranges by endpoints include all numbers subsumed within that range (e.g., 1 to 5 includes 1, 1.5, 2, 2.75, 3, 3.80, 4, 5, etc.).

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

Additional details of these and other embodiments are set forth in the accompanying the description below. Other features, objects and advantages will become apparent from the description and from the claims.

### DETAILED DESCRIPTION

Skin wounds designated as "chronic" or "slow to heal" wounds, "non-healing" wounds or "stalled wounds" and are commonly observed in a clinical setting as venous ulcers, diabetic ulcers, particularly diabetic foot ulcers, or combinations of these two entities. Similar non-healing wounds can be observed in less frequent conditions, e.g., autoimmune disorders but irrespective of the etiology the wounds share the characteristic of failing to heal in a clinically expected timeframe. Empirically, wounds that take longer than 12 weeks to heal from the first observation of a wound are considered to fall into the "chronic" "non-healing", or "slow to heal" categories. The term "stalled wound" also includes some wounds that fail to heal. However, a "stalled wound" is more commonly associated with a wound that begins to heal but, for unknown reasons, stops healing before complete closure of the wound. The fundamental cause(s) of this lack of healing is not well-understood. There exists a need for a treatment for any of these types of wounds since spontaneous healing has failed to occur. The present invention relates to the compositions and wound dressings as described in the claims.

Excess inflammation, which can be concomitant with microbiological infection, can inhibit normal healing processes in a skin wound. The present disclosure relates to compositions, articles, and methods for treating a skin wound. The compositions and articles include an antimicrobial biguanide compound and an active ingredient of inorganic solids comprising salts of potassium, zinc, calcium, and rubidium. The components of the inventive composition surprisingly provide a synergistic effect that results in a suppression of the accumulation of a biochemical marker (e.g., a polypeptide, a cytokine such as interleukin-8(IL-8), for example) associated with inflammation.

Compositions of the present disclosure comprise an active ingredient of inorganic solids. Exemplary active ingredients of inorganic solids of the present disclosure can be found in oak bark ash and related synthetic compositions, as described in U.S. Patent Nos. 6,149,947 and 7,014,870.

The therapeutic activity of various constituents of oak bark extract has been analyzed and the results indicate therapeutic efficacy for compositions containing just potassium, zinc and calcium ions, in combination with suitable counterions. Thus, synthetic formulations contain, by weight of inorganic solids, 10 to 80 parts potassium ions, preferably 30 to 50 parts; 0.00001 to 20 parts zinc ions, preferably 1 to 10 parts; 0.01 to 10 parts calcium ions, preferably 1 to 5 parts; and 0 to 40 parts rubidium ions, preferably 1 to 30 parts; together with pharmaceutically acceptable counterions (e.g., Cl⁻, SO₄⁻, C0₃⁻, OH⁻, Br⁻). Optionally, the composition further may comprise up to 5 parts sulfur, in the form of elemental sulfur or sulfate anions. The solution may also contain other inorganic cations, for example, up to 10 parts by weight of inorganic solids of cobalt, copper, iron, manganese, nickel, strontium or aluminum ions; preferably any of the foregoing can be present in the composition up to 1 part by weight. Further, the composition includes a pharmaceutically acceptable carrier such as water or an ointment or cream base which will result in a therapeutic composition having a pH in the range of 4 to 7, inclusive. Preferably, the composition has a pH in the range of 4.5 to 5.5, inclusive. As used herein, a composition comprising "PHI salts" refers to a composition that includes potassium, zinc, calcium, and rubidium cations together with pharmaceutically-acceptable counterions.

Oak bark extract or the related synthetic mixtures of inorganic solids have been found to provide a variety of beneficial therapeutic properties for the treatment of a variety of skin conditions, as described herein.

Compositions according to the invention are also useful for treating abrasions and other partial thickness wounds. Useful compositions include at least potassium, zinc and calcium ions, for example. The composition is advantageously applied in a cream or ointment base for a period of time (e.g., several hours to several days).

While not intending to be bound by any particular mechanism of action, it appears that oak bark extract and synthetic mixtures containing the key ingredients of oak bark extract function to enhance wound healing by providing complexing ions which interact with biological membranes and/or enzymes including, but not restricted to, alkaline phosphatase, carbonic anhydrase, carboxypeptidase, various enhydrogenases, arginase, camosinase, dehydropeptidase, glycine dipeptidase, histidine deaminase and tripeptidase, oxyloacetic carboxylase, and some lecithinases and enolases. These enzymes are involved in numerous biosynthetic pathways necessary for wound healing, for example, collagen biosynthesis, and are believed to function with greater efficiency in the presence of the complexing ions.

Compositions of the present disclosure further comprise an antimicrobial biguanide compound. The inventive compositions comprise as antimicrobial biguanide compound polyhexamethylene biguanide (PHMB). The use of PHMB in a biosynthesized cellulose wound dressing is
described by Mulder et al. ("Polyhexamethylene Biguanide (PHMB): Antimicrobial Agents in Wound Care"; Wounds; 19(7):173-182; 2007.

Other suitable biguanides include, for example, chlorhexidine and octenidine. Compositions comprising combinations of biguanide compounds are also included.

The biguanide compound is present in the composition in an amount effective to provide antimicrobial activity (e.g., bactericidal and/or bacteristatic activity) in the wound site. Compositions of the present disclosure can comprise 0.1 weight percent to 0.5 weight percent biguanide compound (e.g. polyhexamethylene biguanide).

One or more of the components of the composition can be dissolved and/or suspended in a pharmaceutically-accepted carrier. In any embodiment, the carrier may be water, a gel, or an ointment (e.g., WHITFIELD'S ointment). "Pharmaceutically-acceptable", as used herein refers to a carrier that does not include an ingredient that substantially interferes with the healing process of a wound with which the carrier is contacted. In some embodiments, the carrier may also comprise an aqueous solution or suspension of ethylhexylglycerin, cocamidopropyl hydroxysultaine, lactic acid, sodium hydroxide, glycerin, xylitol, polyvinyl pyrrolidone, polyvinyl pyrrolidone, polyethylene glycol 660 hydroxystearate, or a combination of any two or more of the foregoing. Advantageously, the foregoing pharmaceutically-acceptable carriers can also facilitate the penetration of the compositions into the wound and/or provide local pH changes that may benefit the wound healing.

The present disclosure further provides a wound dressing. The wound dressing comprises a support and any of the compositions disclosed herein wherein the composition includes a pharmaceutically-acceptable carrier; an effective amount of an active ingredient of inorganic solids comprising a potassium salt, a zinc salt, a calcium salt, and a rubidium salt; and a biguanide compound comprising an amount effective to reduce the number of viable microorganisms at a wound site. In some embodiments, the composition can be applied to the dressing as a coating (e.g., a coating on a surface of the dressing that, in use, is oriented toward a wound). In some embodiments, the wound dressing may be imbued (e.g., saturated) with the composition.

Wound dressings of the present disclosure may comprise a variety of support materials including, but not limited to, a fibrous material, a film, a gel, a foam, a hydrocolloid, an alginate, a hydrogel a polysaccharide paste, a plurality of granules, a plurality of beads or a combination of any two or more of the foregoing. In some embodiments, the dressing may further comprise a backing (e.g., an adhesive backing to protect the wound dressing and, optionally, provide adhesive secural of the dressing to a patient's skin).

Herein also described is a method of treating a wound. In some embodiments, the method comprises contacting a wound with any composition of the present disclosure wherein the composition includes a pharmaceutically-acceptable carrier; an effective amount of an active ingredient of inorganic solids comprising a potassium salt, a zinc salt, a calcium salt, and a rubidium salt; and a biguanide compound comprising an amount effective to reduce the number of viable microorganisms at a wound site. The composition can be applied to the wound, for example, in a liquid (e.g., by lavaging the wound with the liquid) or in a gel or an ointment. Liquid compositions can provide substantially immediate delivery of the ions directly to the healing tissue. In contrast, gels and ointments can provide delivery of the ions over a sustained period of time. In some embodiments, the composition can be applied to a wound dressing, which is subsequently contacted with the wound. Advantageously, a dressing comprising the composition can be contacted with the wound for a period of time (e.g., several hours to a day, or longer), thereby providing a moist environment enriched with the PHI cations and the antimicrobial biguanide to facilitate healing of the skin.

### EXAMPLES

Unless otherwise indicated, all parts and percentages are on a weight basis, all water is distilled water, and all molecular weights are weight average molecular weight.

**Materials.** Materials utilized in the preparation of the examples are shown in Tables 1-3.

**Table 1. Materials Table**

| **Component** | **Description** | **Source** |
|---|---|---|
| Normal porcine vaginal mucosa | Full-thickness squamous epithelium | University of Minnesota, St. Paul, MN |
| RPMI-1640 | Cell culture media | Invitrogen Life Technologies; Carlsbad, CA |
| Todd-Hewitt Broth (TH) | Bacteria culture media | Becton Dickinson; Franklin Lakes, NJ |
| Phosphate Buffered Saline (PBS) | Isotonic buffer | Sigma-Aldrich; St. Louis, MO |

**Table 2. PHMB Neutralizer Solution Ingredients**

| **Component** | **Description** | **Source** | **g/L** |
|---|---|---|---|
| Potassium Phosphate, Monobasic | Potassium Phosphate, Monobasic | JT Baker | 0.4 |
| Sodium Phosphate, Dibasic | Sodium Phosphate, Dibasic | Sigma | 10.1 |
| Triton X-100 | Polyethylene glycol *tert-*octylphenyl ether | Aldrich | 1.0 |
| Lecithin | Phosphatidylcholine | Alpha Aesar | 3.0 |
| Tween 80 | Polyethylene glycol sorbitan monooleate | Fluka | 30.0 |
| Sodium Thiosulfate, pentahydrate | Sodium Thiosulfate, pentahydrate | Sigma-Aldrich | 1.0 |
| Water | Water (distilled) | Tap | q.s. to 1 L |

**Table 3. PHMB Gel Ingredients**

| **Component** | **Description** | **Source** | **Weight %** |
|---|---|---|---|
| Cosmocil CQ (20% PHMB) | Polyhexamethylene Biguanide (PHMB) | Arch Chemicals | 2.5 |
| Sensiva SC 50 | Ethylhexylglycerin | Schulke and Mayr | 0.3 |
| Mackam 50 SB | Cocamidopropyl Hydroxysultaine | Rhodia Novecare | 2.5 |
| HiPure 90 | Lactic Acid | Purac | 5 |
| Sodium Hydroxide | Sodium Hydroxide | Spectrum Chemicals | 0.8 |
| Glycerin | Glycerin | Spectrum Chemicals | 5 |
| Xylasorb | Xylitol | Roquette Pharma | 5 |
| PVP-K90 | Polyvinyl Pyrrolidone | BASF | 2 |
| Tylose H 4070 NG | Hydroxyethylcellulose | ShinEtsu | 1 |
| Solutol HS-15 | Polyethylene Glycol 660 Hydroxystearate | BASF | 1 |
| Water | | | 75 |

### MSSA Preparation

Methicillin sensitive *S. aureus* (MSSA, strain MN8, a Toxic Shock Syndrome Toxin 1 (TSST-1⁺)-producing clinical mucosal isolate) was streaked onto an agar plate from frozen stock. From this agar plate, an overnight bacterial culture was prepared in TH broth. The culture was washed and diluted in fresh RPMI 1640 to a concentration of about 5 x 10⁸ CFU/mL.

### Tissue Preparation

Specimens of normal porcine vaginal mucosa were excised from animals at slaughter and rinsed with RPMI-1640. Tissue plugs of uniform size were obtained from the porcine vagina tissue using a 5 mm biopsy punch. Excess muscle tissue was trimmed away with a scalpel. Tissue explants were washed in RPMI-1640 media. The explants were placed, mucosal side up, in a sterile petri dish and washed with fresh RPMI-1640. Explants were incubated at 37C for 30 minutes, then transferred, mucosal side up, to a 6 well plate with 0.4 µm cell culture transwell inserts containing 0.75 mL RPMI-1640.

A pipette was used to apply the diluted MSSA preparation to the mucosal surface of each tissue sample. After incubation for 2 hours at 37C, the infected tissues were removed from the incubator and treated with TEGADERM Matrix (3M Health Care, St. Paul, MN) and/or PHMB Gel.

### PHMB Gel

PHMB gel was prepared with the ingredients in Table 3. Briefly, all of the ingredients, with the exception of hydroxyethylcellulose and glycerol, were mixed with about 80% of the water. This mixture was stirred until clear, and then the pH was adjusted to 3.5 with lactic acid or sodium hydroxide. The remaining water was added to the mixture. The glycerol and hydroxyethylcellulose were mixed to form a slurry and the slurry was added to the aqueous mixture while stirring. The resulting solution was allowed to stir and thicken overnight at room temperature.

### Example 1. Treatment of tissue explant with a dressing comprising an active ingredient of inorganic ions (PHI salts) and a composition comprising a biguanide compound.

Porcine vaginal tissue was infected with MRSA as described above. Ten microliters of PHMB gel were then applied to the tissue. Immediately following application of the PHMB gel, a small square (0.5cm²) of TEGADERM Matrix dressing was applied to the PHMB gel on the tissue. Tissue explants were returned to 37C incubator for 18h. Two separate experiments were run, each experiment using explant material from a different animal. Samples from the separate experiments were subjected to the Microbial Inhibition Test and the Interleukin-8 Accumulation Test (described below) and the results of the separate experiments are shown in Tables 4 and 5, respectively.

### Comparative Example 1. Treatment of tissue explant with a dressing comprising an active ingredient of inorganic ions.

A small square (0.5cm²) of TEGADERM Matrix dressing was applied to the MSSA-infected tissue. The tissue explants were returned to 37C incubator for 18h. Two separate experiments were run, each experiment using explant material from a different animal. Samples from the separate experiments were subjected to the Microbial Inhibition Test and the Interleukin-8 Accumulation Test (described below) and the results of the separate experiments are shown in Tables 4 and 5, respectively.

### Comparative Example 2. Treatment of tissue explant with the PHMB gel.

Porcine vaginal tissue was infected with MRSA as described above. Ten microliters of PHMB gel were then applied to the tissue. After application of the PHMB gel, the tissue explants were returned to 37C incubator for 18h. Samples were subjected to the Microbial Inhibition Test and the Interleukin-8 Accumulation Test (described below) and the results are shown in Table 5.

### Controls

The control for the microbial inhibition test consisted of agar plates, infected as described above, with no PHI and no PHMB applied.

The control for the IL-8 test consisted of vaginal tissue, infected with MRSA as described above, with no PHI and no PHMB applied. A background reading was also recorded, which consisted of IL-8 concentrations from non-infected vaginal tissue.

### Test Methods

### Microbial Inhibition Test

After treatment with TEGADERM Matrix and/or PHMB Gel as described in the Example section and after the incubation step, explants were removed from the transwells and homogenized with 250 µl of PHMB Neutralizer Solution. Serial dilutions of this homogenized tissue were prepared in sterile PBS and plated on Tryptic Soy Agar with 5% sheep blood (Becton Dickinson). After incubating the plates at 37° C for 24 hours, the number of bacterial colonies on each plate was counted and recorded.

### Interleukin-8 (IL-8) Accumulation Test

Samples, prepared as described in the Microbial Inhibition Test, were placed in 250 µl of PBS buffer, homogenized, and centrifuged to clear. The supernatant was transferred to a 1.5 mL tube and frozen until the ELISA analysis. A Porcine IL-8 ELISA Development kit (catalog number DY535; R&D Systems; Minneapolis, MN) was utilized to measure IL-8 in the supernatant from homogenized tissue. Note, because the undiluted samples of PHMB in the PHMB neutralizing solution interfered with the ability to detect IL-8, these samples were diluted 1:4 with sterile water before measuring the IL-8.

**Table 4. Results of the Microbial Inhibition and IL-8 Accumulation tests for first set of tissue explants. All results shown are based on the average value measured in duplicate test samples. The number of micrograms in parentheses refers to the amount of PHMB gel applied to the tissue explants.**

| Sample | Microbial Inhibition* (log₁₀ reduction of CFU/g tissue) | IL-8 Accumulation |
|---|---|---|
| Control | - | 3907 ± 941 |
| Comparative Example 1 | -2.15 | 693 ± 363 |
| Example 1 (1.2 µg) | 4.33 | 16 ± 3 |
| Example 1 (2 µg) | 1.88 | 0 ±0 |
| Example 1 (4 µg) | 1.27 | 18 ± 19 |

| | | |
|---|---|---|
| * Microbial Inhibition is reported as the log₁₀ reduction in the number of MSSA colony-forming units compared to the colony counts on the control plates. | | |

**Table 5. Results of the Microbial Inhibition and IL-8 Accumulation tests for second set of tissue explants. With the exception of the background, all results shown for the microbial inhibition test include the average and standard deviation of duplicate tests.**

| Sample | Microbial Inhibition* (log₁₀ reduction of CFU/g tissue) | IL-8 Accumulation |
|---|---|---|
| Control | - | 4674 ± 1527 |
| Comparative Example 1 | -5.26 | 52 ± 201 |
| Comparative Example 2 | 0.73 | 3740 ± 1428 |
| Example 1 | 2.47 | 0** |

| | | |
|---|---|---|
| * Microbial Inhibition is reported as the log₁₀ reduction in the number of MSSA colony-forming units compared to the colony counts on the control plates. ** The absorbance readings obtained for these samples were less than the background controls and, thus, the IL-98 accumulation is reported as "0". | | |

The results, shown in Tables 4 and 5, indicate that applying compositions comprising the PHI salts with the PHMB resulted in a 1.27-4.33 log₁₀ reduction in the number of bacteria on the infected tissue, compared to the control that was not treated with PHI salts or PHMB. In contrast, infected tissue that was treated only with the PHI salts did not appear to inhibit the bacteria and the infected tissue that was treated only with the PHMB gel resulted in a smaller log₁₀ reduction (0.73) in the number of microorganisms. Furthermore, tissue treated with compositions comprising the PHI salts and PHMB showed lower (i.e., practically undetectable) accumulation of IL-8, compared to tissue treated with PHI salts only or PHMB only.

## Claims

1. A composition comprising:
a pharmaceutically acceptable carrier;
an effective amount of an active ingredient of inorganic solids comprising a potassium salt, a zinc salt, a calcium salt, and a rubidium salt; and
an antimicrobial biguanide compound comprising an amount effective to reduce the number of viable microorganisms at a wound site, wherein the biguanide compound comprises polyhexamethylene biguanide (PHMB);
wherein each of the salts comprises a pharmaceutically acceptable anion.

2. The composition of claim 1, wherein the active ingredient of inorganic solids comprises 10-80 parts of potassium ions, 0.00001-20 parts of zinc ions, 0.01-10 parts of calcium ions and rubidium ions in an amount up to 40 parts, said parts being expressed as parts by weight of inorganic solids.

3. The composition of claim 1 or claim 2, wherein the biguanide compound further comprises chlorhexidine, octenidine, and combinations thereof.

4. The composition of any one of the preceding claims, wherein the composition comprises 0.1 weight percent to 0.5 weight percent of the biguanide compound.

5. The composition of any one of the preceding claims wherein, relative to a composition without the biguanide compound, the composition with the biguanide compound suppresses the accumulation of a polypeptide associated with inflammation.

6. The composition of any one of claims 1 through 4 wherein, relative to a composition without the active ingredient, the composition with the active ingredient suppresses the accumulation of a polypeptide associated with inflammation:

7. The composition of claim 5 or claim 6, wherein the polypeptide associated with inflammation comprises a cytokine.

8. The composition of claim 7, wherein the cytokine comprises interleukin-8.

9. A wound dressing comprising:
the composition of any one of the preceding claims; and
a support.

10. The wound dressing of claim 9, wherein the support comprises a fibrous material, a film, a gel, a foam, a hydrocolloid, an alginate, a hydrogel a polysaccharide paste, a plurality of granules, a plurality of beads or a combination of any two or more of the foregoing.

11. The wound dressing of claim 9 or claim 10, further comprising a backing layer.

12. A composition of any one of claims 1 through 8 for use in treating a wound, comprising contacting a wound with the composition.

13. A wound dressing of any one of claims 9 through 11 for use in treating a wound, comprising contacting a wound with the wound dressing.

## Patentansprüche

1. Eine Zusammensetzung, umfassend:
einen pharmazeutisch verträglichen Träger;
eine wirksame Menge eines Wirkstoffs aus anorganischen Feststoffen, umfassend ein Kaliumsalz, ein Zinksalz, ein Calciumsalz und ein Rubidiumsalz; und
eine antimikrobielle Biguanid-Verbindung, umfassend eine Menge, die wirksam ist, um die Anzahl von lebensfähigen Mikroorganismen an einer Wundstelle zu reduzieren, wobei die Biguanid-Verbindung Polyhexamethylenbiguanid (PHMB) umfasst;
wobei jedes der Salze ein pharmazeutisch verträgliches Anion umfasst.

2. Die Zusammensetzung nach Anspruch 1, wobei der Wirkstoff aus anorganischen Feststoffen 10-80 Teile Kaliumionen, 0,00001-20 Teile Zinkionen, 0,01-10 Teile Calciumionen und Rubidiumionen in einer Menge von bis zu 40 Teilen umfasst, wobei die Teile als Gewichtsteile von anorganischen Feststoffen ausgedrückt sind.

3. Die Zusammensetzung nach Anspruch 1 oder Anspruch 2, wobei die Biguanid-Verbindung ferner Chlorhexidin, Octenidin und Kombinationen davon umfasst.

4. Die Zusammensetzung nach einem der vorhergehenden Ansprüche, wobei die Zusammensetzung 0,1 Gewichtsprozent bis 0,5 Gewichtsprozent der Biguanid-Verbindung umfasst.

5. Die Zusammensetzung nach einem der vorhergehenden Ansprüche, wobei, bezogen auf eine Zusammensetzung ohne die Biguanid-Verbindung, die Zusammensetzung mit der Biguanid-Verbindung die Anhäufung eines mit einer Entzündung in Zusammenhang stehenden Polypeptids unterdrückt.

6. Die Zusammensetzung nach einem der Ansprüche 1 bis 4, wobei, bezogen auf eine Zusammensetzung ohne den Wirkstoff, die Zusammensetzung mit dem Wirkstoff die Anhäufung eines mit einer Entzündung in Zusammenhang stehenden Polypeptids unterdrückt.

7. Die Zusammensetzung nach Anspruch 5 oder Anspruch 6, wobei das mit einer Entzündung in Zusammenhang stehende Polypeptid ein Zytokin umfasst.

8. Die Zusammensetzung nach Anspruch 7, wobei das Zytokin Interleukin-8 umfasst.

9. Ein Wundverband, umfassend:
die Zusammensetzung nach einem der vorhergehenden Ansprüche; und
einen Träger.

10. Der Wundverband nach Anspruch 9, wobei der Träger ein faseriges Material, einen Film, ein Gel, einen Schaum, ein Hydrokolloid, ein Alginat, ein Hydrogel, eine Polysaccharidpaste, eine Mehrzahl von Granalien, eine Mehrzahl von Kügelchen oder eine Kombination aus zwei oder mehreren der vorgenannten umfasst.

11. Der Wundverband nach Anspruch 9 oder Anspruch 10, ferner umfassend eine Trägerschicht.

12. Eine Zusammensetzung nach einem der Ansprüche 1 bis 8 zur Verwendung bei der Behandlung einer Wunde, umfassend in Kontakt bringen einer Wunde mit der Zusammensetzung.

13. Ein Wundverband nach einem der Ansprüche 9 bis 11 zur Verwendung bei der Behandlung einer Wunde, umfassend in Kontakt bringen einer Wunde mit dem Wundverband.

## Revendications

1. Composition comprenant :
un véhicule pharmaceutiquement acceptable ;
une quantité efficace d'un principe actif de solides inorganiques comprenant un sel de potassium, un sel de zinc, un sel de calcium, et un sel de rubidium ; et
un composé antimicrobien biguanide comprenant une quantité efficace pour réduire le nombre de microorganisme viables au niveau d'un site de plaie, lequel composé biguanide comprend du polyhexaméthylène-biguanide (PHMB) ;
dans laquelle chacun des sels comprend un anion pharmaceutiquement acceptable.

2. Composition selon la revendication 1, dans laquelle le principe actif de solides inorganiques comprend 10 à 80 parties d'ions potassium, 0,00001 à 20 parties d'ion zinc, 0,01 à 10 parties d'ions calcium, et des ions rubidium en une quantité allant jusqu'à 40 parties, lesdites parties étant exprimées comme des parties en poids de solides inorganiques.

3. Composition selon la revendication 1 ou la revendication 2, dans laquelle le composé biguanide comprend en outre de la chlorhexidine, de l'octénidine, et leurs combinaisons.

4. Composition selon l'une quelconque des revendications précédentes, laquelle composition comprend de 0,1 % en poids à 0,5 % en poids du composé biguanide.

5. Composition selon l'une quelconque des revendications précédentes, dans laquelle, par rapport à une composition sans le composé biguanide, la composition avec le composé biguanide supprime l'accumulation d'un polypeptide associé à une inflammation.

6. Composition selon l'une quelconque des revendications 1 à 4, dans laquelle, par rapport à une composition sans le principe actif, la composition avec le principe actif supprime l'accumulation d'un polypeptide associé à une inflammation.

7. Composition selon la revendication 5 ou la revendication 6, dans laquelle le polypeptide associé à une inflammation comprend une cytokine.

8. Composition selon la revendication 7, dans laquelle la cytokine comprend de l'interleukine-8.

9. Pansement comprenant :
la composition de l'une quelconque des revendications précédentes ; et
un support.

10. Pansement selon la revendication 9, dans lequel le support comprend un matériau fibreux, un film, un gel, une mousse, un hydrocolloïde, un alginate, un hydrogel, une pâte de polysaccharide, une pluralité de granules, une pluralité de perles, ou une combinaison de deux ou plus de deux quelconques des précédents.

11. Pansement selon la revendication 9 ou la revendication 10, comprenant en outre une couche d'envers.

12. Composition selon l'une quelconque des revendications 1 à 8, pour une utilisation dans le traitement d'une plaie, comprenant la mise en contact d'une plaie avec la composition.

13. Pansement selon l'une quelconque des revendications 9 à 11, pour une utilisation dans le traitement d'une plaie, comprenant la mise en contact d'une plaie avec le pansement.
